# FASCICULE DE BREVET EUROPEEN

(11) **EP 0 390 676 B1**
(45) Date de publication et mention de la délivrance du brevet: **18.01.1995**
(21) Numéro de dépôt: 90400839.8
(22) Date de dépôt: 28.03.1990
(51) Int. Cl.: C12N 15/15, C12N 15/81

(54) **Souche de saccharomyces cerevisiae productrice d'une protéine hétérologue et procédé de préparation de ladite protéine hétérologue par fermentation de ladite souche**
Saccharomyces-cerevisiae-Stamm, der ein heterologes Protein produziert, und Verfahren zur Herstellung dieses heterologen Proteins durch Fermentation dieses Stammes
Saccharomyces cerevisiae strain producing an heterologeous protein and process for preparing said heterologeous protein by fermentation of said strain

(30) Priorité: 31.03.1989 FR 8904306
(43) Date de publication de la demande: 03.10.1990
(73) Titulaire: TRANSGENE S.A., 67082 Strasbourg Cédex (FR)
(72) Inventeur: Lemoine, Yves, F-67100 Strasbourg (FR); Nguyen, Martine, F-67670 Wittersheim (FR); Achstetter, Tilman, D-7602 Oberkirch (DE)
(74) Mandataire: Warcoin, Jacques

(56) Documents cités:
- EP-A- 0 206 783
- EP-A- 0 252 854
- EP-A- 0 273 800
- EP-A- 0 327 797
- EP-A- 0 341 215
- EP-A- 0 349 435
- EUR. J. BIOCHEM. vol. 73, pp. 553-556, Berlin; DIETER H. WOLF et al
- CELL vol. 48, March 1987, pp. 887-897, Cambridge, Mass., US; LUIS A. VALLS et al.
- CHEMICAL ABSTRACTS vol. 98, no. 7, Fevrier 1983, abrege no. 50144 u, Columbus, Ohio , US; E.W. JONES et al.
- NUCLEIC ACID RESEARCH vol. 13,no. 23, 1985, page 8587-8601 Oxford; K. STRUHL:
- JOURNAL OF BACTERIOLOGY vol. 147, no. 2, Aout 1981, page 418-426 Baltimore, US; D.H. WOLF et al.
- FR 88 08978 (document de priorité de EP 349 435)

## Description

La présente invention concerne la préparation de protéines hétérologues, et plus particulièrement d'hirudine, par des souches de levures S. cerevisiae recombinantes. Par protéine hétérologue, on entend une protéine qui n'est ni produite naturellement par la levure, ni nécessaire à sa croissance.

Les levures sont des organismes eucaryotes unicellulaires ; le genre de levure Saccharomyces comprend des souches dont la biochimie et la génétique sont étudiées intensivement en laboratoire ; il comprend également des souches utilisées dans l'industrie alimentaire (pain, boissons alcoolisées ...) et produites par conséquent en très grande quantité. La facilité avec laquelle on peut manipuler la génétique des cellules de Saccharomyces cerevisiae et la longue histoire industrielle de cette espèce en font donc un hôte de choix pour la production de protéines étrangères en utilisant les techniques de l'ADN recombinant.

Lors de la préparation de protéines par les levures recombinantes, on a souvent pu constater que la protéine produite à partir de la souche de levure n'est pas parfaitement homogène, ce qui conduit à des rendements souvents insuffisants pour des protéines d'intérêt industriel destinées à être produites en grande quantité.

La demanderesse s'est déjà particulièrement intéressée á la production d'hirudine par des souches de S. cerevisiae recombinantes, décrites dans des publications européennes de brevet EP-A-0252854 et EP-A-0273800.

L'hirudine dont la source principale se trouve dans les glandes salivaires des sangsues médicinales sous forme d'un mélange de peptides de 65 et 66 acides aminés est un inhibiteur très spécifique et très efficace de la thrombine. Il s'agit donc d'un agent thérapeutique très intéressant dont l'utilisation en clinique exige une très grande pureté du produit, et qui est donc un candidat intéressant à la production par génie génétique.

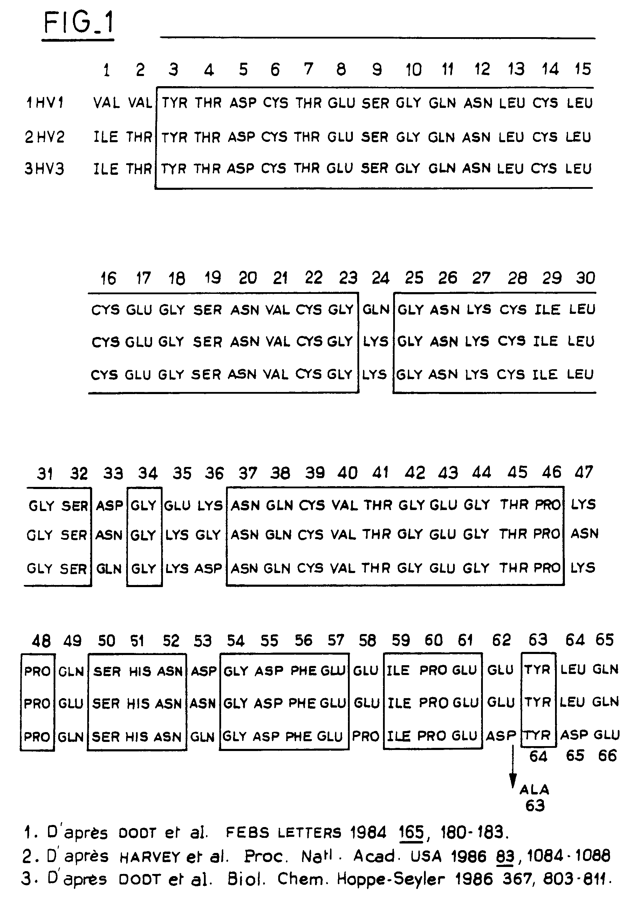

Un certain noire de variants naturels de l'hirudine ont été identifiés et désignés par HV1, HV2, HV3. Leur structure est décrite dans la figure 1. Par la suite ces variants naturels ainsi que d'autres analogues ont été préparés par génie génétique, en particulier par fermentation de souches de S. cerevisiae comme cela est décrit par exemple dans les publications européennes de brevet déjà citées EP-A-0252854 EP-A-0273800 au nom de la Demanderesse.

La demande de brevet EP 341 215 (Ciba Geigy) se rapporte à la suppression de la fonction protéolytique associée à la carboxypeptidase Ysc alpha chez une souche de levure.

La demande de brevet EP 327 797 décrit une souche de Saccharomyces cerevisiae déficiente pour un certain nombre de protéases -dont la protéase A codée par le gène PRA1- et dans laquelle le gène PRC1 est en outre inactivé.

La Demanderesse a observé que l'hirudine produite à partir de la souche de levure décrite dans la publication européenne de brevet EP-A-0252854 n'est pas totalement homogène. En particulier la forme majeure et correcte qui comprend 65 acides aminés est contaminée par deux formes qui éluent d'une colonne HPLC très près du pic principal et qui correspondent à des produits de clivage ayant perdu 1 ou 2 acides aminés à l'extrémité C-terminale. La présence de ces deux formes de 63 et 64 acides aminés rend difficile la purification de la forme correcte à 65 acides aminés.

C'est pourquoi l'invention se propose de fournir de nouveaux moyens pour l'obtention à partir d'une souche de S. cerevisiae d'une plus grande quantité d'hirudine correspondant à la forme correcte, non clivée.

Dans la suite de la description, on emploiera le terme "hirudine" pour désigner toutes les hirudines variants naturels ou analogues ayant subi une ou plusieurs mutations, délétions, tout en conservant leur activité antithrombotique. En effet, l'invention peut concerner la production de toutes les hirudines, même si les exemples ci-après concerneront plus particulièrement l'analogue désigné par rHV2Lys47 (c'est-à-dire un variant HV2 recombinant ayant subi une mutation de l'amino acide Asp en position 47 en amino acide Lys). L'invention peut également concerner toutes les protéines hétérologues produites par des souches de S. cerevisiae recombinantes pour lesquelles on recontre le problème de défaut d'homogénéité du à la présence de formes clivées mentionné plus haut.

L'invention a donc pour objet une souche de S. cerevisiae recombinante productrice d'une protéine hétérologue, transformée par un vecteur d'expression contenant un fragment d'ADN codant pour la protéine hétérologue, caractérisée en ce que le gène PEP4 codant pour la protéase A est Fonctionnel et la fonction protéolytique codée par le gène PRC1 natif a été supprimée.

Les souches de S. cerevisiae présentent un certain nombre de fonctions protéolytiques qui s'expriment, dans la cellule de levure, dans des endroits différents. Il semble que les protéases vacuolaires jouent un rôle particulier dans le défaut d'homogénéité des protéines hétérologues, et en particulier de l'hirudine, produites par des souches de S. cerevisiae.

L'invention repose donc sur la mise en évidence du rôle précis joué par la fonction protéolytique codée par le gène PRC1, et correspondant à une protéase endogène vacuolaire de levure la carboxypeptidase yscY, dans le défaut d'homogénéité de l'hirudine.

La suppression de la fonction protéolytique codée par le gène PRC1 peut être effectuée de différentes façons. On peut citer par exemple une ou plusieurs mutations dans le gène de structure correspondant, et en particulier une délétion partielle ou totale du gène. Pour éviter toute réversion de la mutation, il peut être avantageux de procéder par délétion.

Toutes les souches de S. cerevisiae utilisables industriellement pour la production de protéines hétérologues peuvent faire l'objet de l'invention dès lors qu'elles présentent la déficience indiquée.

D'une manière générale, les souches utilisées peuvent être des souches haploïdes de S. cerevisiae, qui portent la mutation ho, conférant l'hétérothallisme, qui sont de type conjugant alpha, et qui présentent des mutations affectant le gène URA3 ainsi que différents gènes de la biosynthèse d'acides aminés. L'intérêt d'utiliser des souches hétérothalliques est de permettre de conserver la souche sous forme haploïde et non sporulante. En effet, lorsqu'on utilise pour la synthèse d'hirudine le promoteur MFalpha1 de levure, on a constaté que ce promoteur fonctionnait de façon optimale dans les souches non sporulantes de type conjugant alpha. Chez les levures de type sauvage HO, l'haplophase est un état transitoire qui disparaît après une ou deux divisions. Les levures de laboratoire sont des mutants ho chez qui l'haplophase est stable.

Bien entendu, d'autres promoteurs que celui de la phéromone alpha peuvent être utilisés, qui ne requièrent pas nécessairement des souches hétérothalliques. On peut citer par exemple, les promoteurs de levures dont la fonctionalité a été confirmée par la transcription de gènes codant pour des protéines hétérologues : le promoteur PGK, PHO5, GAL1 etc...

L'utilisation de mutants ura3 permet la transformation des souches par un vecteur convenant pour l'expression d'hirudine, ainsi que la sélection de cellules transformées et productrices d'hirudine par la composition du milieu de culture, qui doit être dépourvu de pyrimidine (essentiellement uracile, cytosine, uridine).

Enfin, l'invention concerne également un procédé de préparation d'hirudine par fermentation d'une souche de S. cerevisiae recombinante selon lequel on met en culture une souche de S. cerevisiae selon l'invention dans un milieu approprié et on récupère l'hirudine obtenue.

D'autres caractéristiques et avantages de l'invention apparaîtront au cours de la description détaillée suivante, qui illustre l'invention par des exemples de production du variant rHV2 Lys47 de l'hirudine par une souche de S. cerevisiae selon l'invention accompagnés des figures 1 à 5 suivantes :
- la figure 1 représente les séquences des variants HV1, HV2 et HV3 de l'hirudine.
- la figure 2 représente schématiquement la carte de restriction du gène PRC1 dans sa forme chromosomique et du fragment contenant le gène HIS3 inséré.
- la figure 3 représente la structure du plasmide PTG2958.
- la figure 4 représente le profil HPLC obtenu à partir des surnageants de culture des souches TGY1sp4 et TGY1sp4 prc1-d::HIS3 transformées par le plasmide pTG2958.
- la figure 5 représente le profil HPLC obtenu à partir des surnageants de culture des souches TGY20.3 et TGY20.3 prc1-d::HIS3 transformées par le plasmide pTG2958.
Les caractéristiques génotypiques et phénotypiques des souches de référence et l'historique de leur construction sont détaillés ci-dessous:

### 1. Souche de référence TGY1sp4

- Génotype : MATalpha, ura3-251,-328,-273, his3-11,-15 (le gène URA3 qui est défectif correspond à l'enzyme OMP-décarboxylase ; ce défaut est corrigé en présence d'un plasmide porteur du gène URA3 Cette complémentation constitute le système de sélection du vecteur d'expression de l'hirudine, la souche portant le plasmide pouvant se multiplier en milieu minimum + casaminoacides, sans uracile).
- phénotype : ura- his- ; signe sexuel alpha
- Historique de la souche :
   La souche TGY1sp4 est le résultat du croisement de deux souches de collections, FL ura3 MATa x GRF18 MATalpha.

La souche aFL ura3 a été sélectionnée par F. Lacroute (Université de Strasbourg) comme mutant nécessitant de l'uracile pour la croissance; elle est isogénique de la souche FL100 (souche de l'ATCC, 28383) dont elle dérive et ne se distingue que par le caractère ura- ; ce caractère est dû à trois mutations : ura3-251,-328,-373.

La souche alphaGRF 18 a été sélectionnée par G. Fink (USA) et est utilisée dans de nombreux laboratoires. Son utilisation, dans les manipulations génétiques par transformation, est décrite par exemple par M. Rudolph, I. Koenig-Rauseo et A. Hinnen (Gene 36, 1985, 87-95).

Le génotype de la souche GRF 18 est : MATalpha, his3-11,-15, leu2-3,-112.

### 2. Souche de référence TGY20.3

- Génotype : MATalpha, ura3-251,-373,-328, his3-11,-15, leu2-3,-112, pep4-3.
- Phénoptype : ura-, his-, leu-, croise avec les souches de type MATalpha : ne sporule pas ; présente une importante diminution des activités protéinases yscA, yscB et carboxypeptidase yscY.
- L'historique de la souche est schématisé ci-dessous

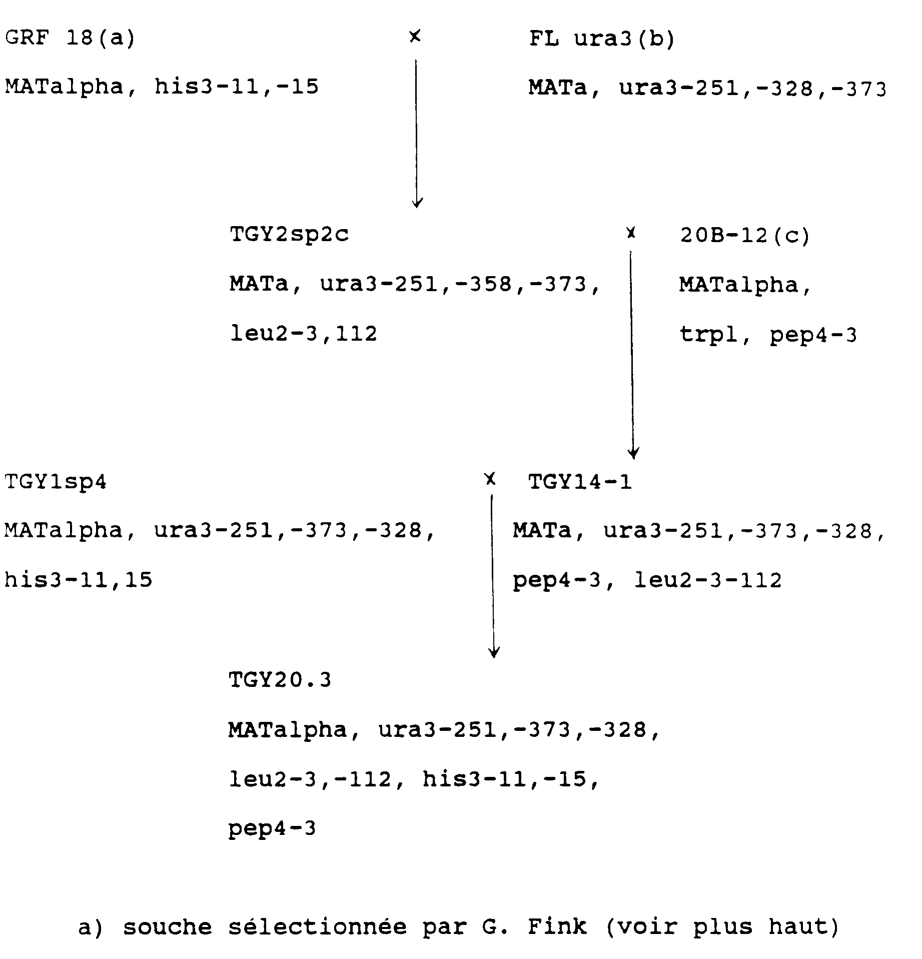

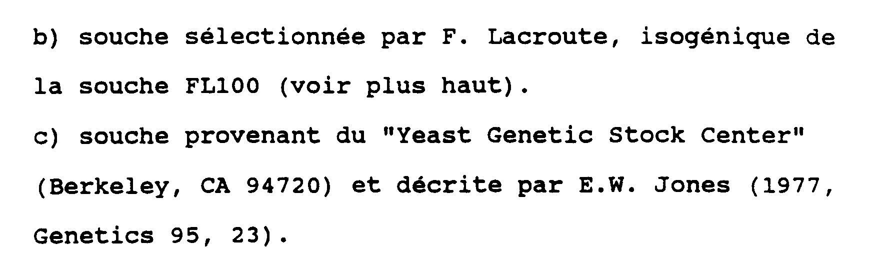

### EXEMPLE 1 : Construction des souches contenant un gène PRC1 patiellement délété

### A. Clonage du gène PRC1

La séquence du gène PRC1 a été publiée par Valls, L.A. et al. [Cell, 48 p 887-897 (1987)]. Deux oligonucléotides sont construits à partir de cette séquence. Le premier est complémentaire de la région 5′ du gène et sa séquence est la suivante :
5′ AAG AAA GAC TGG GAC TTT GTG 3′
Le second est complémentaire de la région 3′ du gène et sa séquence est la suivante :
5′ GAT TGG ATG AAG CCT TAC CAC 3′
A partir d'une banque génomique de levure (fragments d'ADN chromosomique partiellement digéré par Sau3A insérés dans le site BamHI de pFL1 [Parent, S.A. et al. Yeast 1 83-138 (1985)] et par hybridation avec ces deux oligonucléotides on sélectionne un clone de E. coli contenant le gène PRC1 inséré dans pFL1.

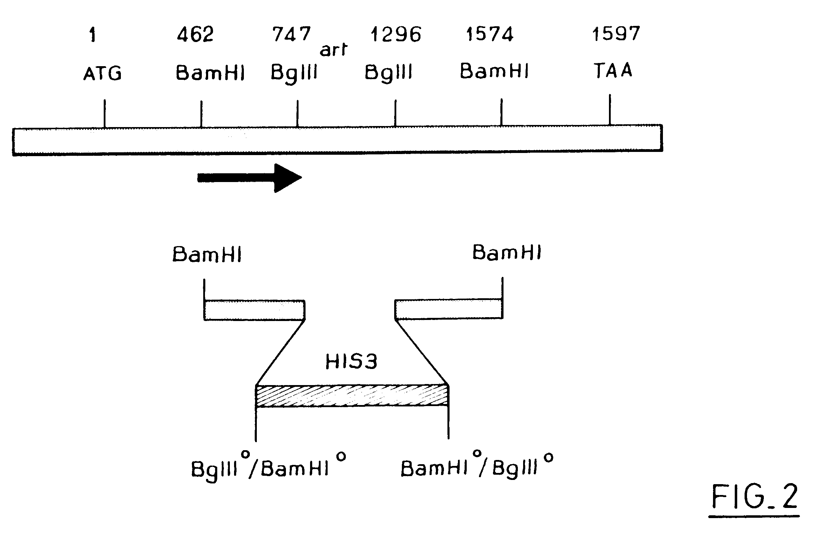

### B. Création de la délétion partielle dans le gène PRC1 (figure 2)

Dans le gène PRC1 on trouve un site BglII dans la séquence codante. Par mutagénèse dirigée un second site BglII est introduit en amont du site BglII naturel à 549 paires de bases de ce dernier c'est à dire toujours dans la séquence codante. Cette mutagénèse est réalisée en utilisant l'oligonucléotide suivant :
5′ CGATGTGGAGATCTTGGCC 3′
Ensuite ce fragment BglII est excisé et remplacé par un fragment BamHI de 1,2 kb contenant la séquence du gène HIS3 de la levure [Struhl K. (1985) Nucl. Acids Res. 13 8587-8601], et obtenu après isolement et clonage dans M13mp8. On élimine ainsi le site actif du produit du gène PRC1.

### C. Sélection des souches contenant le gène muté prc1-d::HIS3 décrit en B

Les souches de S. cerevisiae TGY1sp4 (MATalpha, his3, ura3) et TGY20.3 (MATalpha, his3, ura3, leu2, pep4-3) sont transformées avec un fragment BamHI d'environ 1,7 kb permettant de générer l'allèle muté prc1-d::HIS3 par échange chromosomique. Les clones prototrophes en histidine sont sélectionnés et quatre transformés de chaque souche ensuite analysés pour la stabilité du caractère HIS+. Finalement l'ADN chromosomique de ces transformés est analysé par hybridation de Southern afin de vérifier que l'allèle sauvage PRC1 a bien été échangé avec l'allèle muté prc1-d::HIS3. On obtient ainsi deux souches TGY1sp4 prc1-d::HIS3 et TGY20.3 prc1-d::HIS3.

### EXEMPLE 2 : Construction du plasmide pTG2958

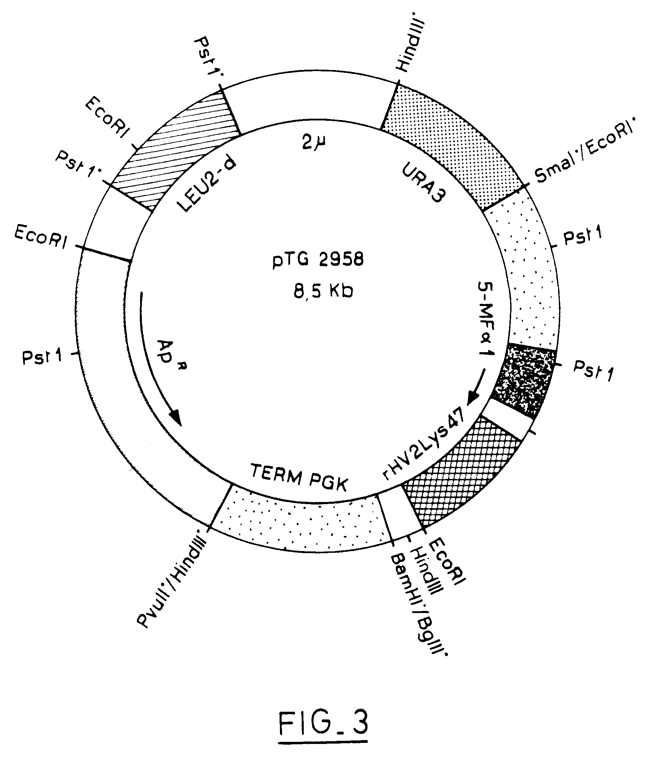

Le plasmide pTG2958 (figure 3) est peu différent du plasmide pTG1833 décrit dans la publication européenne de brevet EP-A-252854 porteur de la séquence codante pour rHV2Asp47. Le plasmide pTG2958 ne contient pas le site de restriction HindIII artificiellement introduit. Le plasmide pTG2958 contient :
- un fragment de 547 paires de bases correspondant à la région 5' du gène MFalpha1 (contenant le promoteur, la séquence codant pour le peptide signal, la région "pro" et une séquence codant pour le peptide Lys-Arg),
- un fragment de 234 paires de bases contenant l'ADN complémentaire de rHV2Lys47,
- un fragment de 243 paires de bases comprenant le terminateur PGK de levure,
- le fragment PvuII-EcoRI de pBR322 comprenant entre autres l'origine de réplication de ce plasmide et le gène de résistance à l'ampicilline (2292 paires de bases),
- le fragment EcoRI-HindIII du plasmide 2» de la levure (forme B), contenant le gène LEU2 de levure, sous forme délétée et inséré dans le site PstI,
- un fragment HindIII-SmaI du gène URA3 de levure.

### EXEMPLE 3 : Production de rHV2Lys47 par les souches précédemment obtenues contenant le gène PRC1 sous une forme délétée

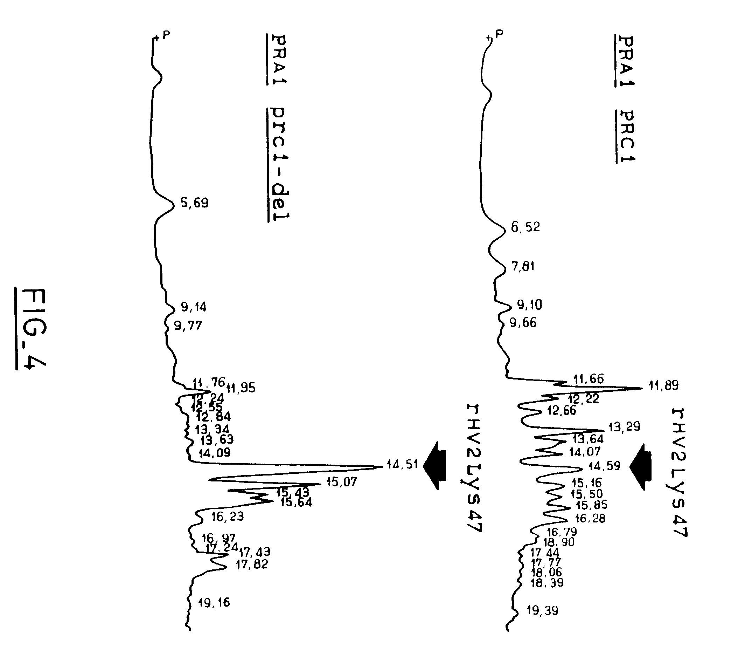

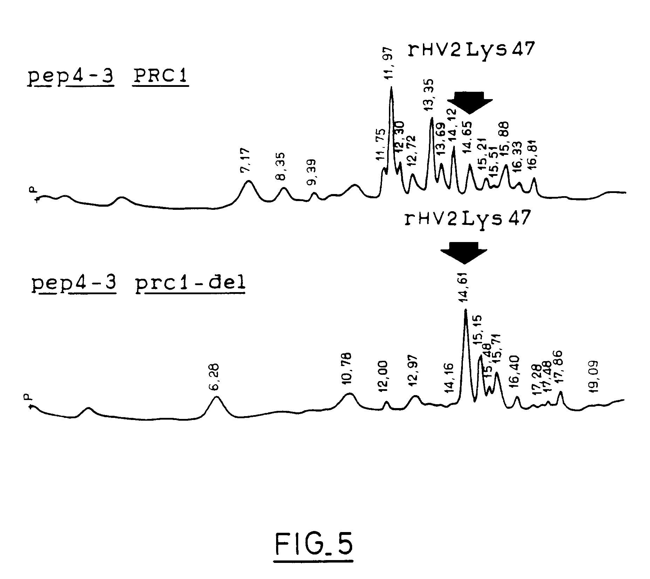

Les souches de levure sont transformées par le plasmide pTG2958 par la méthode de l'acétate de lithium [Ito, H. et al. J. Bactériol. 153; 1983] et les clones prototrophes en uracile sont sélectionnés. Ils sont ensuite mis en culture en erlenmeyer à 30°C sur un milieu sélectif (0,7% de bases azotées pour levures (Yeast Nitrogen Base), 0,5% de casamino acides et 1% de glucose). Après 48 heures de culture, on sépare cellules et surnageant par centrifugation et la quantité de rHV2Lys47 est déterminée dans le surnageant par séparation sur une colonne HPLC (colonne Nucléosil C83»m SFCC N333 équipée d'une précolonne cartouche C8 5»m Nucléosil PSF35-1) et intégration du pic correspondant à la forme 65 acides aminés (figures 4 et 5). Le tableau 1 présente les résultats de ces dosages (les quantités sont données en »g de rHV2Lys47 par unité de densité optique (A600) des cellules obtenues après récolte en phase stationnaire).

**Tableau 1**

| Souche | Génotype Significatif | Production de rHV2Lys47 en »g/A600 |
|---|---|---|
| TGY1sp4 | PRA1 PRC1 | 0,055 |
| - | PRA1 prc1-del::HIS3 | 0,255 |
| TGY20.3 | pep4-3 PRC1 | 0,024 |
| - | pep4-3 prc1-del::HIS3 | 0,072 |

Les dosages de rHV2Lys47 mettent clairement en évidence une augmentation de la production de rHV2Lys47 par un facteur d'environ quatre pour les souches dont le gène PRC1 a été délété selon l'invention. Le dosage immunologique à l'aide d'anticorps monoclonaux spécifiques dirigés contre la partie C-terminale (permettant de discriminer entre les formes dégradées et la forme correcte) confirme ces résultats.
La souche TGY1sp4 prc1-d::HIS3 transformée par le plasmide pTG2958 a été déposée le 31 mars 1989 auprès de la C.N.C.M. 25 rue du Dr. Roux 75724 PARIS sous le numéro I-854.

## Revendications (Revendications pour l'(les) Etat(s) contractant(s) suivant(s): AT, BE, CH, DE, DK, FR, GB, IT, LI, LU, NL, SE)

1. Souche de Saccharomyces cerevisiae recombinante productrice d'une protéine hétérologue, transformée par un vecteur d'expression contenant un fragment d'ADN codant pour la protéine hétérologue, caractérisée en ce que le gène PEP4 codant pour la protéase A est fonctionnel et la fonction protéolytique codée par le gène PRC1 natif a été supprimée.

2. Souche selon la revendication 1 caractérisée en ce que la suppression est obtenue par une mutation opérée sur le gène PRC1.

3. Souche selon la revendication 2 caractérisée en ce que la suppression est obtenue par une délétion dans le gène PRC1.

4. Souche selon la revendication 3 caractérisée en ce qu'un fragment contenant la séquence du gène His3 de la levure est inséré dans le gène prc1-d.

5. Souche selon l'une des revendications 1 à 4 caractérisée en ce que la protéine hétérologue est l'hirudine.

6. Souche selon la revendication 5 caractérisée en ce que la protéine hétérologue est le variant rHV2Lys47 de l'hirudine dans lequel l'acide aminé en position 47 est la Lysine.

7. Procédé de préparation d'hirudine recombinante par fermentation d'une souche de S. cerevisiae caractérisé en ce que l'on met en culture dans un milieu approprié une souche selon l'une des revendications 5 ou 6 et on récupère l'hirudine obtenue.

## Revendications (Revendications pour l'(les) Etat(s) contractant(s) suivant(s): ES, GR)

1. Procédé de préparation d'une souche de Saccharomyces cerevisiae recombinante productrice d'une protéine hétérologue, dans lequel on transforme, par un vecteur d'expression contenant un fragment d'ADN codant pour la protéine hétérologue, une souche de S. cerevisiae caractérisée en ce que le gène PEP4 codant pour la protéase A est fonctionnel et la fonction protéolytique codée par le gène PRC1 natif a été supprimée.

2. Procédé selon la revendication 1 caractérisé en ce que la suppression est obtenue par une mutation opérée sur le gène PRC1.

3. Procédé selon la revendication 2 caractérisé en ce que la suppression est obtenue par une délétion dans le gène PRC1.

4. Procédé selon la revendication 3 caractérisé en ce qu'un fragment contenant la séquence du gène His3 de la levure est inséré dans le gène prc1-d.

5. Procédé selon l'une des revendications 1 à 4 caractérisé en ce que la protéine hétérologue est l'hirudine.

6. Procédé selon la revendication 5 caractérisé en ce que la protéine hétérologue est le variant rHV2Lys47 de l'hirudine dans lequel l'acide aminé en position 47 est la Lysine.

7. Procédé de préparation d'hirudine recombinante par fermentation d'une souche de S. cerevisiae caractérisé en ce que l'on met en culture dans un milieu approprié une souche obtenue par un procédé selon l'une des revendications 5 ou 6 et on récupère l'hirudine obtenue.

## Claims (Claims for the following Contracting State(s): AT, BE, CH, DE, DK, FR, GB, IT, LI, LU, NL, SE)

1. Recombinant strain of Saccharomyces cerevisiae productive of a heterologous protein, transformed by an expression vector containing a DNA fragment coding for the heterologous protein, characterized in that the PEP4 gene coding for the protease A is functional and the proteolytic function encoded by the native PRC1 gene has been suppressed.

2. Strain according to Claim 1, characterized in that the suppression is obtained by a mutation performed on the PRC1 gene.

3. Strain according to Claim 2, characterized in that the suppression is obtained by a deletion in the PRC1 gene.

4. Strain according to Claim 3, characterized in that a fragment containing the sequence of the His3 gene of the yeast is inserted into the prc1-d gene.

5. Strain according to one of Claims 1 to 4, characterized in that the heterologous protein is hirudin.

6. Strain according to Claim 5, characterized in that the heterologous protein is the hirudin variant rHV2Lys47 in which the amino acid at position 47 is lysine.

7. Process for preparing recombinant hirudin by fermentation of an S. cerevisiae strain, characterized in that a strain according to one of Claims 5 or 6 is cultured in a suitable medium and the hirudin obtained is recovered.

## Claims (Claims for the following Contracting State(s): ES, GR)

1. Process for preparing a recombinant strain of Saccharomyces cerevisiae productive of a heterologous protein, in which an S. cerevisiae strain is transformed by an expression vector containing a DNA fragment coding for the heterologous protein, characterized in that the PEP4 gene coding for the protease A is functional and the proteolytic function encoded by the native PRC1 gene has been suppressed.

2. Process according to Claim 1, characterized in that the suppression is obtained by a mutation performed on the PRC1 gene.

3. Process according to Claim 2, characterized in that the suppression is obtained by a deletion in the PRC1 gene.

4. Process according to Claim 3, characterized in that a fragment containing the sequence of the His3 gene of the yeast is inserted into the prc1-d gene.

5. Process according to one of Claims 1 to 4, characterized in that the heterologous protein is hirudin.

6. Process according to Claim 5, characterized in that the heterologous protein is the hirudin variant rHV2Lys47 in which the amino acid at position 47 is lysine.

7. Process for preparing recombinant hirudin by fermentation of an S. cerevisiae strain, characterized in that a strain obtained by a process according to one of Claims 5 or 6 is cultured in a suitable medium and the hirudin obtained is recovered.

## Patentansprüche (Patentansprüche für folgende(n) Vertragsstaat(en): AT, BE, CH, DE, DK, FR, GB, IT, LI, LU, NL, SE)

1. Stamm von Saccharomyces cerevisiae-Rekombinante, der ein heterologes Protein produziert, transformiert durch einen Expressions-Vektor, der ein DNA-Fragment enthält, das für das heterologe Protein codiert, dadurch gekennzeichnet, daß das für die Protease A codierende Gen PEP4 funktionell ist und die proteolytische Funktion, codiert durch das natürliche Gen PRC1, aufgehoben wurde.

2. Stamm nach Anspruch 1, dadurch gekennzeichnet, daß die Aufhebung durch eine an dem Gen PRC1 vorgenommene Mutation erhalten wird.

3. Stamm nach Anspruch 2, dadurch gekennzeichnet, daß die Aufhebung durch eine Deletion in dem Gen PRC1 erhalten wird.

4. Stamm nach Anspruch 3, dadurch gekennzeichnet, daß ein Fragment, das die Sequenz des Gens His3 der Hefe enthält, in das Gen prc1-d eingebracht wird.

5. Stamm nach einem der Ansprüche 1 bis 4, dadurch gekennzeichnet, daß das heterologe Protein Hirudin ist.

6. Stamm nach Anspruch 5, dadurch gekennzeichnet, daß das heterologe Protein die Variante rHV2Lys47 von Hirudin ist, worin die Aminosäure in Position 47 Lysin ist.

7. Verfahren zur Herstellung von Hirudin-Rekombinante durch Fermentation eines Stammes von S. cerevisiae, dadurch gekennzeichnet, daß man in einem geeigneten Milieu einen Stamm gemäß einem der Ansprüche 5 oder 6 in Kultur bringt und das erhaltene Hirudin gewinnt.

## Patentansprüche (Patentansprüche für folgende(n) Vertragsstaat(en): ES, GR)

1. Verfahren zur Herstellung eines Stammes von Saccharomyces cerevisae-Rekombinante, der ein heterologes Protein produziert, bei dem man durch einen Expressions-Vektor, der ein DNA-Fragment enthält, das für das heterologe Protein codiert, einen Stamm von S. cerevisiae transformiert, dadurch gekennzeichnet, daß das für die Protease A codierende Gen PEP4 funktionell ist und die proteolytische Funktion, codiert durch das natürliche Gen PRC1, aufgehoben wurde.

2. Verfahren nach Anspruch 1, dadurch gekennzeichnet, daß die Aufhebung durch eine an dem Gen PRC1 vorgenommene Mutation erhalten wird.

3. Verfahren nach Anspruch 2, dadurch gekennzeichnet, daß die Aufhebung durch eine Deletion in dem Gen PRC1 erhalten wird.

4. Verfahren nach Anspruch 3, dadurch gekennzeichnet, daß ein Fragment, das die Sequenz des Gens His3 der Hefe enthält, in das Gen prc1-d eingebracht wird.

5. Verfahren nach einem der Ansprüche 1 bis 4, dadurch gekennzeichnet, daß das heterologe Protein Hirudin ist.

6. Verfahren nach Anspruch 5, dadurch gekennzeichnet, daß das heterologe Protein die Variante rHV2Lys47 von Hirudin ist, worin die Aminosäure in Position 47 Lysin ist.

7. Verfahren zur Herstellung von Hirudin-Rekombinante durch Fermentation eines Stammes von S. cerevisiae, dadurch gekennzeichnet, daß man in einem geeigneten Milieu einen gemäß dem Verfahren nach einem der Ansprüche 5 oder 6 erhaltenen Stamm in Kultur bringt und das erhaltene Hirudin gewinnt.
